(19) Europäisches Patentamt
European Patent Office
Office européen des brevets

(11) Publication number: **0 177 030**
**A1**

# (12) EUROPEAN PATENT APPLICATION

(21) Application number: 85112466.9

(22) Date of filing: 02.10.85

(51) Int. Cl.[4]: C 07 H 17/08
C 07 D 313/00
//A61K31/71

(30) Priority: 02.10.84 JP 206919/84

(43) Date of publication of application:
09.04.86 Bulletin 86/15

(84) Designated Contracting States:
CH DE FR GB IT LI

(71) Applicant: DAIKIN INDUSTRIES, LIMITED
No. 1-12-39, Umeda Kita-ku
Osaka-shi Osaka-fu(JP)

(72) Inventor: Takahara, Takao
3-12-2, Todaiji Shimanoto-cho
Mishima-gun Osaka-fu(JP)

(72) Inventor: Shimokawa, Kazuhiro
18-2, Shinzaike 2-chome
Settsu-shi Osaka-fu(JP)

(74) Representative: Eitle, Werner, Dipl.-Ing. et al,
Hoffmann, Eitle & Partner Patentanwälte Arabellastrasse 4
D-8000 München 81(DE)

## (54) Process for preparing 8-fluoroerythronolide.

(57) 8-Fluoroerythronolide derivative such as 8-fluoroerythronolide A and 8-fluoroerythromycin is safely prepared by reacting an erythronolide derivative with acylhypofluorite of the formula:

RCOOF (I)

wherein R is a $C_1$-$C_5$ aliphatic group.

EP 0 177 030 A1

# PROCESS FOR PREPARING 8-FLUOROERYTHRONOLIDE

## FIELD OF THE INVENTION

The present invention relates to a process for preparing a 8-fluoroerythronolide derivative. More particularly, it relates to a process for preparing 8-fluoroerythronolide A and 8-fluoroerythromycin.

## BACKGROUND OF THE INVENTION

8-Fluoroerythromycin A is chemically more stable than erythromycin A under an acidic condition and has as broad antibacterial spectrum as that of erythromycin A. Therefore, 8-fluoroerythromycin A is useful for oral administration (cf. Journal of Antibiotics, 36, No. 11, 1585 (1983)).

8-Fluoroerythromycin A has been prepared by reacting 8,9-anhydroerythronolide A-6,9-hemiketal with trifluoromethylhypofluorite to fluorinate the 8-position of the hemiketal and then microbiologically attaching desosamine and cladinose to the 8-fluorocompound (cf. Tetrahedron, 40, 2177 (1984)), or by reacting 8,9-anhydroerythromycin A-6,9-hemiketal-N-oxide with trifluoromethylhypofluorite or perchlorylfluoride to fluorinate the 8-position of the hemiketal and reducing the 8-fluorocompound with hydrogen (cf. Japanese Patent Kokai Publication (unexamined) No. 109496/1983).

In the above processes, trifluoromethylhypofluorite or perchlorylfluoride is used as a fluorination agent, but the reaction system for carrying out the fluorination with these compounds tends to explode. Therefore, the above conventional methods are not suitable for commercial production of 8-fluoroerythromycin A.

## SUMMARY OF THE INVENTION

One object of the present invention is to provide a safe process for preparing 8-fluoroerythronolide derivatives.

Another object of the present invention is to provide a process for preparing 8-fluoroerythronolide derivatives, particularly 8-fluoroerythronolide A and 8-fluoroerythromycin A-N-oxide.

## DETAILED DESCRIPTION OF THE INVENTION

According to the present invention, there is provided a process for preparing a 8-fluoroerythronolide derivative comprising reacting an erythronolide derivative with acylhypofluorite of the formula:

$$RCOOF \qquad (I)$$

wherein R is a $C_1$-$C_5$ aliphatic group.

The 8-fluoroerythronolide derivative has 8,9-anhydro-6,9-hemiketal structure in the molecule and includes, for example, 8,9-anhydroerythronolide A-6,9-hemiketal of the formula:

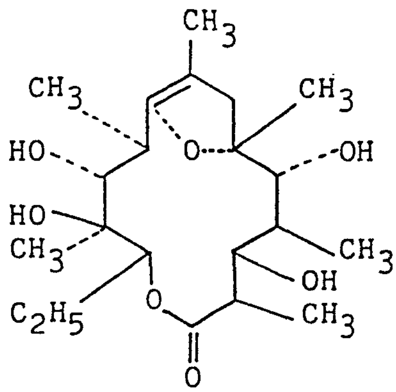

(II)

8,9-anhydroerythromycin A-6,9-hemiketal of the formula:

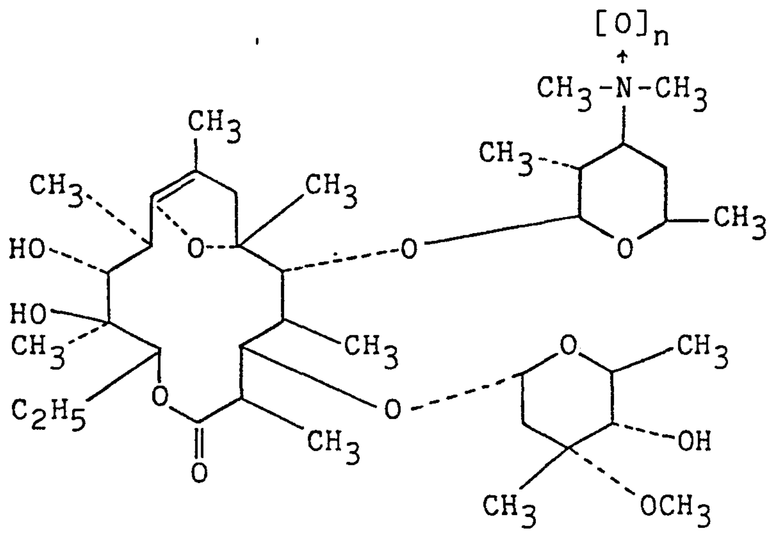

(III)

wherein n is 0 or 1.

The acylhypofluorite may be prepared by reacting an alkali metal salt of a $C_1$-$C_5$ aliphatic acid with fluorine (cf. J. Chem. Soc. Com., (1982) 730). For example, sodium acetate is reacted with fluorine gas.

Specific examples of the 8-fluoroerythronolide derivatives are 8-fluoroerythronolide of the formula:

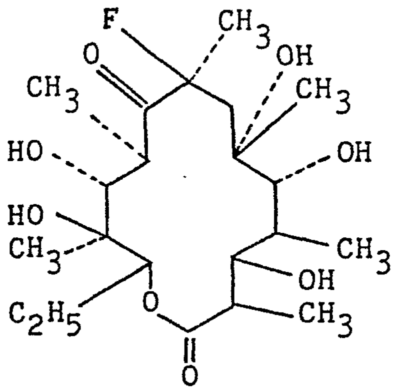

(IV)

and 8-fluoroerythromycin derivatives of the formula:

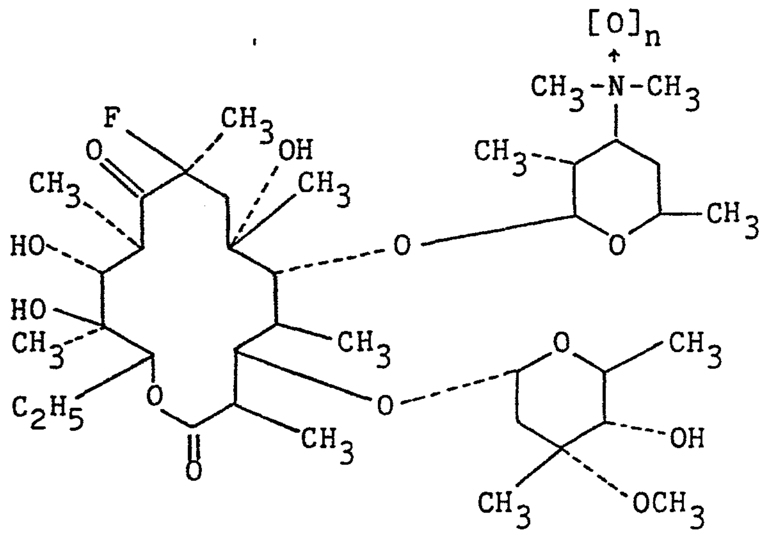

(V)

wehrein n is the same as defined above, which correspond to the erythronolide derivatives (II) and (III), respectively.

Generally, the above reaction according to the present invention is carried out by suspending the alkali metal salt of the aliphatic acid in a chlorofluorocarbon type solvent and/or the $C_1$-$C_5$ aliphatic acid, bubbling fluorine gas diluted with an inert gas such as nitrogen to 1-20 % by mole in the suspension at a temperature of -100 to 0°C, preferably -80 to -60°C to form acylhypofluorite (I) and then adding the reaction mixture in a solution of the

erythronolide derivative in a chlorofluorocarbon type solvent or a chlorocarbon type solvent at a temperature of -100 to 0°C, preferably -80 to -60°C. Usually, a molar ratio of acylhypofluorite (I) and the erythronolide derivative is from 10:1 to 1:1, preferably from 3:1 to 1:1.

Specific examples of the chlorofluorocarbon type solvent are $CCl_3F$, $CCl_2F_2$, $CBrF_3$, $CHCl_2F$, $CHClF_2$, $CClF_2$-$CClF_2$, $CBr_2F_2$ and the like.

Specific examples of the chlorocarbon type solvent are $CH_2Cl_2$, $CHCl_3$ and $CCl_4$.

When the mixture of the chlorofluorocarbon type solvent and the aliphatic acid is used, their volume ratio is not critical. Preferably, the volume ratio of the former and the latter is from 30:1 to 7:1.

Specific examples of the inert gas used for diluting fluorine gas are nitrogen, helium, argon and the like.

8-Fluoroerythronolide A and 8-fluoroerythromycin A-N-oxide are converted into an antibiotics, 8-fluoroerythromycin A as explained above.

The present invention will be hereinafter explained further in detail by following examples.

Example 1

(a) Preparation of $CH_3COOF$

In a 200 ml polyethylene vessel, $CCl_3F$ (100 ml), acetic acid (4 ml) and sodium acetate fine powder (1.2 g, 14.6 mmol) were charged and mixed and then fluorine gas (18 mmol) diluted with nitrogen gas to 10 % by mole was bubbled in the mixture at a rate of 100 ml/min. with vigorously stirring on a dry ice-acetone bath.

(b) Fluorination

To a solution of 8,9-anhydroerythronolide A-6,9-hemiacetal (400 mg, 0.01 mol) in methylene chloride (10 ml) cooled on a dry ice-acetone bath, the reaction mixture prepared in the step (a) was dropwise added. In the course of the reaction, aliquots of the reaction mixture were sampled and analyzed by high pressure liquid chromatography (C-18 radial pack column for reverse phase manufactured by Waters; Solvent, 0.2 M $NH_4OAc/CH_3OH$ (volume ratio of 1:4); Flow rate, 1.0 ml/min.; Detection wavelength, 215 nm) and the reaction proceeded till the peaks due to the raw material disappeared.

After the completion of the reaction, a saturated aqueous solution of sodium hydrogencarbonate (50 ml) was added to the reaction mixture and stirred at a room temperature for one hour. After separating the organic layer, the aqueous layer was extracted with methylene chloride (150

ml). The combined organic solution was evaporated and concentrated. Then, the product was isolated by means of a silica gel column (Wakogel C-200, 30 mm x 1.5 mm) using a mixture of methylene chloride and methanol (volume ratio, 95:5) as an eluent to give 8-fluoroerythronolide A (80 mg). Yield 20 %.

UV spectrum: max 287 nm.

$^1H$-NMR: δ (ppm) = 4.63 (d, $H_{11}$), 4.06 (d, d, $H_3$) and 3.89 (d, $H_5$).

Example 2

To a solution of 8,9-anhydroerythromycin A-6,9-hemiacetal-N-oxide (3.66 g, 5 mmol) in methylene chloride (200 ml), the mixture containing $CH_3COOF$ (50 ml) prepared in Example 1(a) was added at -30°C. The proceeding of the reaction was monitored in the same way as in Example 1(b).

After the completion of the reaction, the reaction mixture was washed with a saturated aqueous solution of sodium hydrogencarbonate and concentrated in the same manner as in Example 1(b) to obtain a crude crystalline product, which was recrystallized from a mixture of methanol and ethyl ether (volume ratio 1:4) to give 8-fluoroerythromycin A-N-oxide (3 g). Yield 82 %. M.P. 168-169°C.

UV spectrum: max 286 nm.

$[\alpha]_D^{20}$ = -6.33°.

CLAIMS:

1. A process for preparing a 8-fluoroerythronolide derivative comprising reacting an erythronolide derivative with acylhypofluorite of the formula:

RCOOF (I)

wherein R is a $C_1$-$C_5$ aliphatic group.

2. A process according to claim 1, wherein the erythronolide derivative is one selected from the group consisting of 8,9-anhydroerythronolide A-6,9-hemiketal and 8,9-anhydroerythromycin A-6,9-hemiketal.

3. A process according to claim 1, wherein the acylhypofluorite is acetylhypofluorite.

4. A process according to claim 1, wherein the reaction temperature is from -100 to 0°C.

5. A process according to claim 4, wherein the reaction temperature is from -80 to -60°C.

6. A process according to claim 1, which is carried out in the presence of a solvent.

7. A process according to claim 6, wherein the solvent is one selected from the group consisting of a chlorofluorocarbon type solvent and a chlorocarbon type solvent.

8. A process according to claim 1, wherein the molar ratio of the acylhypofluorite (I) and the erythronolide derivative is from 10:1 to 1:1.

9. A process according to claim 8, wherein the molar ratio is from 3:1 to 1:1.

European Patent Office

# EUROPEAN SEARCH REPORT

0177030 Application number

EP 85112466.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP - A1 - 0 080 763 (PIERREL)<br>* Abstract; claims 1,2,6,7 *<br>-- | 1,2,6,7 | C 07 H 17/08<br>C 07 D 313/00<br>//A 61 K 31/71 |
| D,Y | TETRAHEDRON, vol. 40, no. 11, 1984, Pergamon Press<br>L. TOSCANO et al. "Preparation of (8S)-8-Fluoroerythronolide A..." pages 2177--2181<br>* Pages 2177,2179 *<br>-- | 1-9 | |
| D,Y | JOURNAL OF THE CHEMICAL SOCIETY CHEMICAL COMMUNICATIONS 1982,<br>M.J. ADAM "...Reaction of Glycals with Hypofluorite" pages 730,731<br>* Totality *<br>---- | 1-9 | |
| | | | TECHNICAL FIELDS SEARCHED (Int. Cl.4) |
| | | | C 07 H 17/00<br>C 07 D 313/00 |

The present search report has been drawn up for all claims

| Place of search | Date of completion of the search | Examiner |
|---|---|---|
| VIENNA | 12-12-1985 | JANISCH |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document